# EUROPEAN PATENT APPLICATION

(11) **EP 4 818 203 A1**
(43) Date of publication of application: **07.10.2026**
(21) Application number: 24915765.2
(22) Date of filing: 19.04.2024
(51) Int. Cl.: C07K 16/12, G01N 33/569

(54) **DIAGNOSTIC KIT FOR SKIN COMPRISING ANTIBODY FOR LACTOBACILLUS PLANTARUM LDH1**

(30) Priority: 04.01.2024 KR 20240001780
(71) Applicant: Kolmar Korea Co., Ltd., Sejong 30004 (KR)
(72) Inventor: KIM, Jin Kyoung, Seoul 06800 (KR); SON, Hee Seung, Seoul 06800 (KR); JUN, Ji Hyun, Seoul 06800 (KR); BAE, Hyung Jin, Seoul 06800 (KR); KIM, Jin Mo, Seoul 06800 (KR); HAN, Sang Keun, Seoul 06800 (KR)
(74) Representative: Nederlandsch Octrooibureau
(86) International application number: PCT/KR2024/005347
(87) International publication number: WO 2025/146874

(57) **Abstract**

The present invention relates to an antibody specific to *Lactobacillus plantarum* **LDH1,** and to a composition or kit comprising the same for detecting *Lactobacillus plantarum* or diagnosing skin conditions. The monoclonal antibody according to the present invention is highly reactive to antigens, and, particularly, can detect antigens rapidly and accurately even at low concentrations due to two antibodies of different CDRs having been combined and used as capture and detection antibodies, thus allowing the monoclonal antibody to be used in rapid self-diagnostic kits and the like. In using the monoclonal antibody, the degree of proliferation of *Lactobacillus plantarum* on the skin surface and the like can be visually checked, abnormal states of the skin can be diagnosed, the incidence probability and persistence of related diseases can be predicted, and subsequent treatment methods can be determined.

## Description

### TECHNICAL FIELD

The present invention relates to an antibody specific to *Lactobacillus plantarum* LDH1, and to a composition or kit for detecting *Lactobacillus plantarum* or diagnosing skin conditions, comprising the same.

### BACKGROUND ART

Recently, as research on the microbiome has been actively conducted, the fields of gut microbiome and skin microbiome have been gaining attention. Among these, in the case of the skin microbiome, very detailed results and data can be obtained using methods such as NGS and genetic testing, but there is no rapid diagnostic method capable of conveniently checking the skin condition.

Meanwhile, recent studies have shown that as one ages, the diversity of the skin microbiome increases and the abundance of *Lactobacillus* and *Cutibacterium* decreases, suggesting that the skin microbiome may be related to skin aging. Among these, lactic acid bacteria (LAB) are generally known as probiotics, and related human studies are being actively conducted. Although the skin application of lactic acid bacteria is still in its early stages compared to oral intake, various beneficial effects on the skin are being revealed. In particular, *Lactobacillus plantarum* is known to be involved in alleviating symptoms of atopic dermatitis in adults through its immunomodulatory effect, and there are also research results showing its effects on psoriasis, acne, and seborrheic dermatitis.

Accordingly, the present inventors endeavored to develop a kit for detecting *Lactobacillus plantarum*, which can have a positive effect on skin health. In particular, in a state where consumer accessibility to rapid diagnostic kits has greatly increased due to COVID, the present inventors sought to develop a rapid self-diagnostic kit for measuring the skin surface concentration of LDH1, a major protein of *Lactobacillus plantarum*. The present inventors developed novel monoclonal antibodies against the LDH1, selected the optimal combination thereof, and confirmed that *Lactobacillus plantarum* can be rapidly and accurately detected, thereby completing the present invention.

### DETAILED DESCRIPTION OF THE INVENTION

### TECHNICAL PROBLEM

An object of the present invention is to provide an antibody specific to the LDH1 protein of *Lactobacillus plantarum*, and a composition or kit for detecting *Lactobacillus plantarum* comprising the same.

The technical tasks of the present invention are not limited to the above-mentioned technical tasks, and other technical tasks that are not mentioned will be clearly understood by those skilled in the art from the description below.

### TECHNICAL SOLUTION

According to an embodiment of the present invention, there is provided an antibody specific to the LDH1 protein of *Lactobacillus plantarum*, comprising a heavy chain variable region comprising heavy chain CDR1 represented by the amino acid sequence of SEQ ID NO: 1, heavy chain CDR2 represented by the amino acid sequence of SEQ ID NO: 2, and heavy chain CDR3 represented by the amino acid sequence of SEQ ID NO: 3; and a light chain variable region comprising light chain CDR1 represented by the amino acid sequence of SEQ ID NO: 4, light chain CDR2 represented by the amino acid sequence of SEQ ID NO: 5, and light chain CDR3 represented by the amino acid sequence of SEQ ID NO: 6.

In addition, there is provided an antibody specific to the LDH1 protein of *Lactobacillus plantarum*, comprising a heavy chain variable region comprising heavy chain CDR1 represented by the amino acid sequence of SEQ ID NO: 9, heavy chain CDR2 represented by the amino acid sequence of SEQ ID NO: 10, and heavy chain CDR3 represented by the amino acid sequence of SEQ ID NO: 11; and a light chain variable region comprising light chain CDR1 represented by the amino acid sequence of SEQ ID NO: 12, light chain CDR2 represented by the amino acid sequence of SEQ ID NO: 13, and light chain CDR3 represented by the amino acid sequence of SEQ ID NO: 14.

### ADVANTAGEOUS EFFECTS

The monoclonal antibody according to the present invention is a novel antibody specific to the LDH1 protein of *Lactobacillus plantarum*, and has very high reactivity to the antigen. In particular, the monoclonal antibody according to the present invention can detect antigens rapidly and accurately even at low concentrations by combining two antibodies having different CDRs as a capture antibody and a detection antibody, and thus can be applied to rapid self-diagnostic kits and the like. In addition, by using the same, the proliferation state of *Lactobacillus plantarum* on the skin surface and the like can be intuitively confirmed, so that skin conditions can be diagnosed, the incidence probability and persistence of related diseases can be predicted, and subsequent treatment methods can be determined.

### DESCRIPTION OF THE DRAWINGS

To help understand the drawings in the detailed description of the present invention more sufficiently, a brief description of each drawing is provided.
FIG. 1 shows the SDS-PAGE results confirming the purified monoclonal antibodies.
FIG. 2 shows the results of comparing the affinity of the selected monoclonal antibodies and a commercialized antibody for the LDH1 antigen through indirect ELISA (A: Biorbyt (orb847579)).
FIG. 3 shows the results of comparing the affinity of the selected monoclonal antibodies and a commercialized antibody for the LDH1 antigen through Western blot.
FIG. 4 shows the results of observing the reactivity to the LDH1 antigen of the monoclonal antibody combinations according to the present invention on a rapid kit strip.
FIG. 5 shows the results of observing the reactivity to the LDH1 antigen on a rapid kit using the monoclonal antibodies 2H1 and 3D5 according to the present invention.
FIG. 6 shows the results of confirming that *Lactobacillus plantarum* can be detected when an actual skin sample is applied to a rapid kit manufactured using the monoclonal antibodies 2H1 and 3D5 according to the present invention.

### MODES OF THE INVENTION

According to an embodiment of the present invention, there is provided an antibody specific to the LDH1 protein of *Lactobacillus plantarum*, comprising a heavy chain variable region comprising heavy chain CDR1 represented by the amino acid sequence of SEQ ID NO: 1, heavy chain CDR2 represented by the amino acid sequence of SEQ ID NO: 2, and heavy chain CDR3 represented by the amino acid sequence of SEQ ID NO: 3; and a light chain variable region comprising light chain CDR1 represented by the amino acid sequence of SEQ ID NO: 4, light chain CDR2 represented by the amino acid sequence of SEQ ID NO: 5, and light chain CDR3 represented by the amino acid sequence of SEQ ID NO: 6.

In addition, there is provided an antibody specific to the LDH1 protein of *Lactobacillus plantarum*, comprising a heavy chain variable region comprising heavy chain CDR1 represented by the amino acid sequence of SEQ ID NO: 9, heavy chain CDR2 represented by the amino acid sequence of SEQ ID NO: 10, and heavy chain CDR3 represented by the amino acid sequence of SEQ ID NO: 11; and a light chain variable region comprising light chain CDR1 represented by the amino acid sequence of SEQ ID NO: 12, light chain CDR2 represented by the amino acid sequence of SEQ ID NO: 13, and light chain CDR3 represented by the amino acid sequence of SEQ ID NO: 14.

According to another embodiment of the present invention, there is provided a polynucleotide encoding the antibody.

According to still another embodiment of the present invention, there is provided an expression vector comprising the polynucleotide.

According to still another embodiment of the present invention, there is provided a host cell comprising the expression vector.

According to still another embodiment of the present invention, there is provided a composition for detecting *Lactobacillus plantarum* comprising the antibody.

According to still another embodiment of the present invention, there is provided a kit for detecting *Lactobacillus plantarum* comprising the antibody.

Unless defined otherwise, all technical and scientific terms used herein have the same meaning as commonly understood by one of ordinary skill in the art to which the invention pertains. Generally, the nomenclature used herein is well known and commonly used in the art. Furthermore, when describing embodiments of the present invention, when it is determined that detailed descriptions of related known configurations or functions would hinder understanding of the embodiments of the present invention, the detailed descriptions will be omitted. Furthermore, while embodiments of the present invention will be described below, the technical spirit of the present invention is not limited or restricted thereto, and may be modified and implemented in various ways by those skilled in the art.

When a part is said to "comprise" a certain component, unless specified otherwise, this means that it may further include other components, rather than exclude other components. The term "and/or" as used herein encompasses a combination of a plurality of related items or any one of a plurality of related items.

According to an embodiment of the present invention, there is provided a novel antibody specific to the LDH1 protein of *Lactobacillus plantarum*.

In the present invention, the term "antibody" refers to a protein molecule that serves as a receptor specifically recognizing an antigen, including an immunoglobulin molecule that has immunological reactivity with a specific antigen, and includes both polyclonal antibodies, monoclonal antibodies, whole antibodies, and antibody fragments.

In the present invention, the term "monoclonal antibody" refers to an antibody molecule of single molecular composition obtained from a substantially identical antibody population, and such a monoclonal antibody exhibits single binding specificity and affinity for a specific epitope.

For the purposes of the present invention, the antibody may be a monoclonal antibody that specifically binds to the LDH1 protein of *Lactobacillus plantarum*.

In the present invention, an immunoglobulin has a heavy chain and a light chain, and each of the heavy and light chains comprises a constant region and a variable region (the regions are also known as domains). The variable regions of the light and heavy chains comprise three hypervariable regions called complementarity-determining regions (hereinafter referred to as "CDRs") and four framework regions. The CDRs primarily serve to bind to an epitope of an antigen. The CDRs of each chain are sequentially called CDR1, CDR2, and CDR3 starting from the N-terminus, and are also identified by the chain in which the specific CDR is located.

According to an embodiment of the present invention, there is provided an antibody specific to the LDH1 protein of *Lactobacillus plantarum*, comprising a heavy chain variable region comprising heavy chain CDR1 represented by the amino acid sequence of SEQ ID NO: 1, heavy chain CDR2 represented by the amino acid sequence of SEQ ID NO: 2, and heavy chain CDR3 represented by the amino acid sequence of SEQ ID NO: 3; and a light chain variable region comprising light chain CDR1 represented by the amino acid sequence of SEQ ID NO: 4, light chain CDR2 represented by the amino acid sequence of SEQ ID NO: 5, and light chain CDR3 represented by the amino acid sequence of SEQ ID NO: 6, and this antibody was named 2H1.

The antibody may comprise a heavy chain variable region represented by the amino acid sequence of SEQ ID NO: 7, and a light chain variable region represented by the amino acid sequence of SEQ ID NO: 8.

According to another embodiment of the present invention, there is provided an antibody specific to the LDH1 protein of *Lactobacillus plantarum*, comprising a heavy chain variable region comprising heavy chain CDR1 represented by the amino acid sequence of SEQ ID NO: 9, heavy chain CDR2 represented by the amino acid sequence of SEQ ID NO: 10, and heavy chain CDR3 represented by the amino acid sequence of SEQ ID NO: 11; and a light chain variable region comprising light chain CDR1 represented by the amino acid sequence of SEQ ID NO: 12, light chain CDR2 represented by the amino acid sequence of SEQ ID NO: 13, and light chain CDR3 represented by the amino acid sequence of SEQ ID NO: 14, and this antibody was named 3D5.

The antibody may comprise a heavy chain variable region represented by the amino acid sequence of SEQ ID NO: 15, and a light chain variable region represented by the amino acid sequence of SEQ ID NO: 16.

The antibody of the present invention may comprise variants of the amino acid sequences described in the attached sequence listing within the range that can specifically recognize the LDH1 protein of *Lactobacillus plantarum*. For example, changes may be made to the amino acid sequence of the antibody to improve the binding affinity and/or other biological properties of the antibody. Such modifications include, for example, deletion, insertion, and/or substitution of amino acid sequence residues of the antibody. Such amino acid variations are made based on the relative similarity of amino acid side-chain substituents, such as hydrophobicity, hydrophilicity, charge, and size. Through analysis of the size, shape, and type of amino acid side-chain substituents, it can be seen that arginine, lysine, and histidine are all positively charged residues; alanine, glycine, and serine have similar sizes; and phenylalanine, tryptophan, and tyrosine have similar shapes. Therefore, based on these considerations, arginine, lysine, and histidine; alanine, glycine, and serine; and phenylalanine, tryptophan, and tyrosine can be said to be biologically functional equivalents.

In introducing variations, the hydropathic index of amino acids may be considered. Each amino acid is assigned a hydropathic index according to its hydrophobicity and charge: Isoleucine (+4.5); Valine (+4.2); Leucine (+3.8); Phenylalanine (+2.8); Cysteine/Cystine (+2.5); Methionine (+1.9); Alanine (+1.8); Glycine (-0.4); Threonine (-0.7); Serine (-0.8); Tryptophan (-0.9); Tyrosine (-1.3); Proline (-1.6); Histidine (-3.2); Glutamate (-3.5); Glutamine (-3.5); Aspartate (-3.5); Asparagine (-3.5); Lysine (-3.9); and Arginine (-4.5).

The hydrophobic amino acid index is very important in conferring interactive biological function to proteins. It is a well-known fact that substitution must be made with amino acids having a similar hydropathic index in order to retain similar biological activity. When variations are introduced with reference to the hydropathic index, substitution is made between amino acids exhibiting hydropathic index differences preferably within ±2, more preferably within ±1, and even more preferably within ±0.5.

Meanwhile, it is also well known that substitution between amino acids having similar hydrophilicity values results in proteins with equivalent biological activity. The following hydrophilicity values are assigned to each amino acid residue: Arginine (+3.0); Lysine (+3.0); Aspartate (+3.0 ± 1); Glutamate (+3.0 ± 1); Serine (+0.3); Asparagine (+0.2); Glutamine (+0.2); Glycine (0); Threonine (-0.4); Proline (-0.5 ± 1); Alanine (-0.5); Histidine (-0.5); Cysteine (-1.0); Methionine (-1.3); Valine (-1.5); Leucine (-1.8); Isoleucine (-1.8); Tyrosine (-2.3); Phenylalanine (-2.5); and Tryptophan (-3.4).

When variations are introduced with reference to the hydrophilicity value, substitution is made between amino acids exhibiting hydrophilicity value differences preferably within ±2, more preferably within ±1, and even more preferably within ±0.5.

In addition, amino acid exchanges in proteins that do not overall alter the activity of the molecule are known in the art (H. Neurath, R.L. Hill, The Proteins, Academic Press, New York, 1979). The most commonly occurring exchanges are between the amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly.

Accordingly, the antibody according to the present invention may have 80 to 99% homology, 90 to 99% homology, or 95 to 99% homology to the above-described amino acid sequences.

According to another embodiment of the present invention, there is provided a polynucleotide encoding the antibody. The polynucleotide includes not only natural nucleotides but also analogues in which the sugar or base moiety is modified. The polynucleotide may be modified, and the modification includes addition, deletion, or non-conservative substitution or conservative substitution of nucleotides.

According to still another embodiment of the present invention, there is provided an expression vector comprising the polynucleotide and a host cell comprising the expression vector.

In the present invention, the term "vector" is a means for expressing a target gene in a host cell, and includes plasmid vectors; cosmid vectors; and viral vectors such as bacteriophage vectors, adenovirus vectors, retrovirus vectors, and adeno-associated virus vectors. In the vector of the present invention, the polynucleotide encoding the antibody may be operatively linked to a promoter. The term "operatively linked" refers to a functional linkage between a nucleic acid expression control sequence (e.g., an array of promoter or transcription regulatory factor binding sites) and another nucleic acid sequence, whereby the regulatory sequence controls the transcription and/or translation of the other nucleic acid sequence. The vector system of the present invention can be constructed through various methods known in the art, and typically can be constructed as a vector for cloning or a vector for expression. In addition, the vector of the present invention can be constructed using a prokaryotic cell or a eukaryotic cell as a host.

Meanwhile, the expression vector of the present invention may include an antibiotic resistance gene commonly used in the art as a selection marker, and the antibiotic resistance gene may be at least one selected from ampicillin, gentamicin, carbenicillin, chloramphenicol, streptomycin, kanamycin, geneticin, neomycin, and tetracycline.

In addition, in the present invention, the host cell may be a bacterial or animal cell. Cells transformed with the vector are host cells capable of stably and continuously cloning and expressing the vector of the present invention, and any host cell known in the art may be used. For example, suitable eukaryotic host cells for the vector include, but are not limited to, monkey kidney cells (COS7), NSO cells, SP2/0, Chinese hamster ovary (CHO) cells, W138, baby hamster kidney (BHK) cells, MDCK, myeloma cell lines, HuT 78 cells, and HEK-293 cells.

According to still another embodiment of the present invention, there is provided a composition or kit for detecting *Lactobacillus plantarum* comprising the antibody. The composition or kit may be for diagnosing skin conditions.

The composition or kit according to the present invention can detect *Lactobacillus plantarum* by detecting an antigen-antibody complex, and the antigen may comprise the LDH1 protein of *Lactobacillus plantarum* or the strain itself. The antibody may be one or more depending on the detection method. For example, when there are two antibodies, one antibody may be a capture antibody and the other antibody may be a detection antibody. In an embodiment of the present invention, a rapid antigen diagnostic kit was manufactured using the 2H1 antibody as the capture antibody and the 3D5 antibody as the detection antibody.

In the present invention, the "antigen-antibody complex" refers to a binding product of the corresponding protein antigen in a sample and an antibody recognizing the same. The detection of the antigen-antibody complex can be performed using methods known in the art, for example, spectroscopic, photochemical, biochemical, immunochemical, electrical, absorbance, chemical, and other methods. Specifically, the detection can be performed by any method selected from the group consisting of colorimetric method, electrochemical method, fluorimetric method, luminometry, particle counting method, visual assessment, and scintillation counting method. Methods that may be used include, but are not limited to, Western blotting, ELISA (enzyme linked immunosorbent assay), Radioimmunoassay, Radioimmunodiffusion, Ouchterlony immunodiffusion, Rocket immunoelectrophoresis, tissue immunostaining, immunoprecipitation assay, complement fixation assay, FACS, and protein chip. Various labels may be used in the present invention to detect the antigen-antibody complex. Specific examples include enzymes, fluorescent substances, ligands, luminescent substances, microparticles, and radioisotopes, and include but are not limited to colloidal gold particles or beads of colored glass or plastic (e.g., polystyrene, polypropylene, latex).

In the present invention, the kit may be an ELISA (Enzyme-linked immunosorbent assay) kit, a sandwich ELISA kit, a protein chip kit, or a rapid kit, and specifically may be a sandwich ELISA kit or a rapid kit using two antibodies. However, the kit is not limited thereto, and is applicable to all kits using one or more antibodies.

In the present invention, "ELISA (Enzyme-linked immunosorbent assay)" is also called the enzyme immunoassay method, and is a method of quantifying an antigen-antibody complex formed by binding an enzyme to an antibody by using absorbance through the reaction of the enzyme with a substrate. The ELISA includes direct ELISA using a labeled antibody that recognizes an antigen attached to a solid support, indirect ELISA using a labeled secondary antibody that recognizes the capture antibody in a complex of antibodies recognizing an antigen attached to a solid support, direct sandwich ELISA using another labeled antibody that recognizes an antigen in a complex of an antibody attached to a solid support and the antigen, and indirect sandwich ELISA using a labeled secondary antibody that recognizes the antibody after reacting another antibody that recognizes the antigen in a complex of an antibody attached to a solid support and the antigen.

In the present invention, the "rapid kit" is also called Rapid diagnostic test (RDT), rapid antigen test, or immunochromatography kit assay. The rapid kit assay is an analysis method using an immunochromatography strip comprising a first sample pad, a membrane, and an absorbent pad, by which a user can simply detect an analyte from a biological or chemical sample within a short time without special skills or equipment. The antibody according to the present invention has superior reactivity with antigens compared to commercialized antibodies, and thus has excellent sensitivity and specificity, and is characterized by being applicable to a rapid kit that detects the presence of antigens within a short time. The rapid kit may enable detection within, for example, 60 minutes, specifically within 30 minutes, and more specifically within 15 minutes, but is not limited thereto.

The sample used in the kit may include tissue, whole blood, urine, and saliva, but for the purposes of the present invention, may specifically be skin tissue. For example, the sample may be prepared by scraping the surface of the skin tissue with a stick and dissolving it in a solution. Specifically, the kit according to the present invention can be used to rapidly and conveniently self-diagnose the presence and concentration of *Lactobacillus plantarum* proliferating on the skin by directly utilizing the skin surface, i.e., the epidermal tissue. More specifically, the kit can be used as a rapid self-diagnostic kit for diagnosing skin conditions and predicting the incidence probability and persistence of related diseases, and determining subsequent treatment methods by measuring the skin surface concentration of *Lactobacillus plantarum*, which is known to be involved in preventing skin aging and alleviating symptoms of skin diseases including atopic dermatitis.

Hereinafter, examples are presented to explain the present invention more specifically, but the present invention is not limited thereto.

### Example 1. Preparation of monoclonal antibodies against Lactobacillus plantarum LDH1

### 1-1. Immunization of mice

150 µg of LDH1 protein in PBS was mixed with an equal volume of Freund's adjuvant (Incomplete, Sigma) to make a total volume of 600 µl, and then 200 µl per mouse was injected into 6-week-old female BALB/c mice to perform the first immunization. Two weeks later, 150 µg of LDH1 was dissolved in PBS to a total volume of 600 µl, and 200 µl per mouse was injected into the mice that had received the first immunization in the same manner to perform the second immunization.

### 1-2. Cell fusion

Four days after the second immunization, the lymph nodes of the mice were aseptically removed and washed twice with DMEM medium. The washed lymph nodes were made into single cells using a cell strainer (Falcon), then washed again with DMEM and suspended in DMEM. The lymph node cells and SP2/0 cells were mixed at a ratio of 5:1, and 1 ml of PEG (Polyethylene Glycol 1500, Sigma) was slowly added over 2 minutes to induce cell fusion. DMEM medium was added thereto, and after allowing to stand at 37°C for 15 minutes, the supernatant was removed by centrifugation at 1200 rpm. The cells were suspended at a concentration of 1×10⁶ cells/ml in DMEM supplemented with HAT (0.1 mM hypoxanthine, 0.4 M aminopterin, 16 µM thymidine, Sigma) and 20% FBS, and then 100 µl of this suspension was dispensed into a 96-well plate and cultured in a cell incubator for 2 weeks to select fused cells.

### 1-3. Confirmation of antibody formation in fused cells

Antibody production by fused cells was observed by the Indirect ELISA method using the immune antigen and the culture supernatant of the fused cells. The immune antigen was diluted to a concentration of 0.5 µg/ml, and 50 µl was added to each well, and allowed to stand overnight in refrigeration to coat the antigen. Then, each well was washed 3 times with 0.05% PBST. 180 µl of 1% BSA/PBS was added to each well and reacted at room temperature for 1 hour for blocking. 50 µl of the culture supernatant of the fused cells was added to each well, reacted at room temperature for 1 hour, and then washed 3 times with 0.05% PBST. HRP-conjugated anti-mouse IgG antibody (Sigma) was diluted at a ratio of 1:10000 and dispensed at 50 µl per well, and reacted at room temperature for 30 minutes, followed by washing 3 times with 0.05% PBST. TMB substrate (Surmodics) was added at 50 µl per well, and reacted at room temperature for 15 minutes while protected from light. 1N sulfuric acid was added at 50 µl per well to stop the reaction, and the OD (optical density) was measured using an ELISA reader.

### 1-4. Purification of antibodies

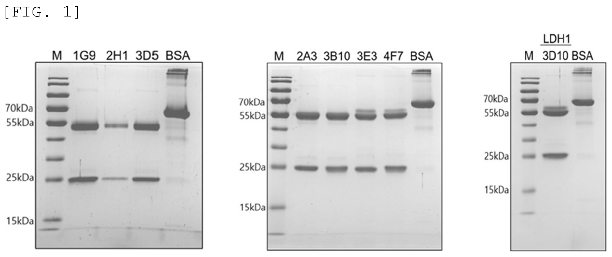

The fused cells producing the antibodies were transferred to a 25T flask for large-scale culture, and the antibodies were purified from the obtained cell supernatant using a Protein G affinity column. After separating a total of 8 antibodies, the purified antibodies were loaded onto a gel to perform SDS-PAGE. The results are shown in FIG. 1.

As shown in FIG. 1, it was confirmed that the antibodies were well purified by confirming the expression of 8 monoclonal antibodies (1G9, 2H1, 3D5, 2A3, 3B10, 3E3, 4F7, 3D10).

### 1-5. Selection of monoclonal antibody candidates

Antibodies were selected by examining the antigen-antibody reactivity using the Sandwich ELISA method. The Sandwich ELISA was performed as follows. First, the total of 8 monoclonal antibodies purified in Example 1-4 were each diluted in carbonate buffer to a concentration of 1 µg/ml, then 100 µl was added to each well and allowed to stand overnight in refrigeration to coat the antibody. After washing each well 3 times with 0.05% PBST, 300 µl of 1% BSA/PBS was added to all wells and reacted at room temperature for 1 hour for blocking. The immune antigen was diluted to concentrations of 1 µg/ml, 0.1 µg/ml, and 0.01 µg/ml, added at 100 µl per well, and reacted at room temperature for 1 hour, then washed 3 times with 0.05% PBST. Each biotinylated antibody was diluted to a concentration of 1 µg/ml, added at 100 µl per well, and reacted at room temperature for 1 hour. After washing 3 times with 0.05% PBST, SA-HRP (Sigma) was diluted at a ratio of 1:8000, added at 100 µl per well, and reacted at room temperature for 30 minutes. After washing in the same manner as above, TMB (Surmodics) was added at 100 µl per well, and reacted at room temperature for 15 minutes while protected from light, after which 1N sulfuric acid was added at 50 µl per well to stop the reaction, and the OD (optical density) was measured using an ELISA reader. By the above method, the monoclonal antibody candidates were selected through the concentration response of antigens and antibodies. The results are shown in Table 1.

**[Table 1]**

| **OD** | **1G9** | **2A3** | **2H1** | **3B10** | **3D5** | **3E3** | **4F7** | **3D10** |
|---|---|---|---|---|---|---|---|---|
| 1000ng/ ml | | 0.139 | 0.188 | 0.299 | 0.109 | 0.172 | 0.216 | 0.679 |
| 100ng/m l | | 0.082 | 0.081 | 0.099 | 0.068 | 0.077 | 0.097 | 0.146 |
| 10ng/ml | | 0.068 | 0.064 | 0.066 | 0.062 | 0.064 | 0.067 | 0.074 |
| B | | 0.070 | 0.061 | 0.063 | 0.059 | 0.059 | 0.064 | 0.066 |

| **OD** | **1G9_1ug/ml** | | | | | | | |
|---|---|---|---|---|---|---|---|---|
| | **1G9** | **2A3** | **2H1** | **3B10** | **3D5** | **3E3** | **4F7** | **3D10** |
| 1000ng/ ml | 0.217 | 0.224 | | 0.790 | 0.317 | 0.261 | 0.478 | 1.107 |
| 100ng/m l | 0.081 | 0.087 | | 0.197 | 0.093 | 0.084 | 0.157 | 0.227 |
| 10ng/ml | 0.081 | 0.087 | | 0.072 | 0.062 | 0.060 | 0.070 | 0.093 |
| B | 0.057 | 0.056 | | 0.058 | 0.060 | 0.057 | 0.057 | 0.079 |

| | **2H1_1ug/ml** | | | | | | | |
|---|---|---|---|---|---|---|---|---|
| **OD** | **1G9** | **2A3** | **2H1** | **3B10** | **3D5** | **3E3** | **4F7** | **3D10** |
| 1000ng/ ml | 0.686 | 0.867 | 1.846 | | 1.285 | 0.844 | 2.016 | 3.873 |
| 100ng/m l | 0.134 | 0.150 | 0.334 | | 0.210 | 0.143 | 0.449 | 0.722 |
| 10ng/ml | 0.065 | 0.076 | 0.092 | | 0.085 | 0.066 | 0.100 | 0.132 |
| B | 0.057 | 0.062 | 0.064 | | 0.074 | 0.060 | 0.059 | 0.070 |

| | **3B10_1ug/ml** | | | | | | | |
|---|---|---|---|---|---|---|---|---|
| **OD** | **1G9** | **2A3** | **2H1** | **3B10** | **3D5** | **3E3** | **4F7** | **3D10** |
| 1000ng/ ml | 0.107 | 0.115 | 0.190 | 0.473 | | 0.110 | 0.288 | 1.856 |
| 100ng/m l | 0.061 | 0.064 | 0.070 | 0.110 | | 0.059 | 0.091 | 0.256 |
| 10ng/ml | 0.050 | 0.053 | 0.052 | 0.055 | | 0.051 | 0.056 | 0.071 |
| B | 0.048 | 0.049 | 0.049 | 0.064 | | 0.049 | 0.053 | 0.053 |

| **OD** | **3D5_1ug/ml** | | | | | | | |
|---|---|---|---|---|---|---|---|---|
| | **1G9** | **2A3** | **2H1** | **3B10** | **3D5** | **3E3** | **4F7** | **3D10** |
| 1000ng/ ml | 0.273 | 0.367 | 0.492 | 0.955 | 0.239 | | 0.710 | 1.956 |
| 100ng/m l | 0.131 | 0.120 | 0.145 | 0.213 | 0.103 | | 0.206 | 0.377 |
| 10ng/ml | 0.113 | 0.094 | 0.089 | 0.094 | 0.085 | | 0.095 | 0.175 |
| B | 0.098 | 0.117 | 0.088 | 0.083 | 0.084 | | 0.083 | 0.163 |

| | **3E3_1ug/ml** | | | | | | | |
|---|---|---|---|---|---|---|---|---|
| **OD** | **1G9** | **2A3** | **2H1** | **3B10** | **3D5** | **3E3** | **4F7** | **3D10** |
| 1000ng/ ml | 0.707 | 0.766 | 1.612 | 2.803 | 0.998 | 0.830 | | 3.728 |
| 100ng/m l | 0.156 | 0.161 | 0.304 | 0.518 | 0.196 | 0.177 | | 0.725 |
| 10ng/ml | 0.091 | 0.099 | 0.106 | 0.127 | 0.113 | 0.095 | | 0.176 |
| B | 0.087 | 0.086 | 0.096 | 0.083 | 0.095 | 0.088 | | 0.115 |

| | **4F7_1ug/ml** | | | | | | | |
|---|---|---|---|---|---|---|---|---|
| **OD** | **1G9** | **2A3** | **2H1** | **3B10** | **3D5** | **3E3** | **4F7** | **3D10** |
| 1000ng/ ml | 0.127 | 0.139 | 0.279 | 0.473 | 0.160 | 0.154 | 0.303 | |
| 100ng/m l | 0.061 | 0.062 | 0.083 | 0.111 | 0.064 | 0.062 | 0.091 | |
| 10ng/ml | 0.053 | 0.055 | 0.058 | 0.057 | 0.053 | 0.053 | 0.054 | |
| B | 0.053 | 0.109 | 0.052 | 0.051 | 0.052 | 0.051 | 0.052 | |
| | **3D10_1ug/ml** | | | | | | | |

As shown in Table 1, as a result of selecting two of the 8 monoclonal antibodies in pairs and performing Sandwich ELISA, it was confirmed that they showed high reactivity to the antigen, and among them, 2H1, 3B10, 3D5, 3D10, and 4F7 were confirmed to show high OD values.

### Example 2. Comparison of affinity for LDH1 antigen between selected monoclonal antibodies and a commercialized antibody

### 2-1. Comparison through Indirect ELISA method

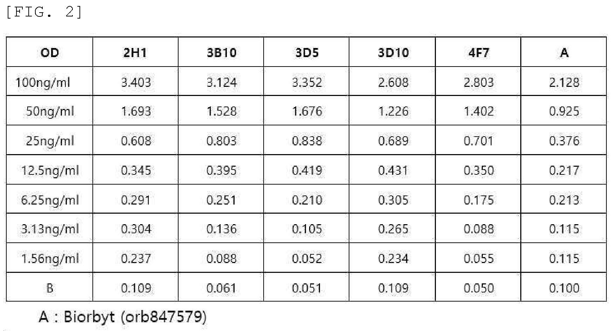

The LDH1 antigen was serially diluted in carbonate buffer to concentrations of 10, 5, 2.5, 1.25, 0.625, 0.313, and 0.156 ng/ml, and 100 µl was added to each well, and allowed to stand overnight in refrigeration to coat the antigen. After washing all wells 3 times with 0.05% PBST, 300 µl of 1% BSA/PBS was added to each well and reacted at room temperature for 1 hour for blocking. After washing all wells 3 times with 0.05% PBST in the same manner as above, the monoclonal antibodies selected in Example 1-5 and the purchased Biorbyt (orb847579) antibody were diluted in 1% BSA/PBS to a concentration of 1 µg/ml, 100 µl was added to each well and reacted at room temperature for 1 hour, then washed 3 times with 0.05% PBST. HRP-conjugated anti-mouse IgG antibody (Sigma) was dispensed at 100 µl per well at a ratio of 1:10000 and reacted at room temperature for 30 minutes, then washed 3 times with 0.05% PBST. TMB substrate (Surmodics) was added at 50 µl per well, and reacted at room temperature for 15 minutes while protected from light. 1N sulfuric acid was added at 50 µl per well to stop the reaction, and the OD (optical density) was measured using an ELISA reader. The results are shown in FIG. 2.

As shown in FIG. 2, the monoclonal antibodies 2H1, 3B10, 3D5, 3D10, and 4F7 selected through the present invention showed superior reactivity compared to the commercialized antibody, and in particular, 2H1 and 3D5 were confirmed to show excellent results.

### 2-2. Comparison through Western blot

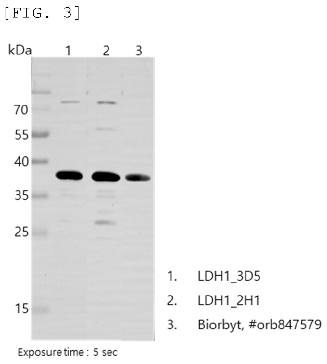

2 µg of LDH1 antigen was added to SDS sample buffer and heated at 95~100°C for 10 minutes, then 20 µl each of the sample and marker were loaded onto a 12% SDS-PAGE gel and electrophoresed at 80~120V for 2 hours. After completion, the gel was removed and rinsed with running water, and placed on a plastic pad for transfer in the order of 3M paper-PVDF-gel-3M paper. It was mounted in a transfer chamber and transferred at 185 mA for about 2 hours. After blocking with 5% skim milk, the monoclonal antibody selected in Example 1 above and the purchased Biorbyt (orb847579) antibody were reacted as primary antibodies at a ratio of 1:1000 in refrigeration overnight. After washing three times with 1x TBST for 5 minutes each, it was reacted with a secondary antibody at a ratio of 1:4000 for 1 hour at room temperature, and washed three times with 1x TBST for 5 minutes each. ECL buffer was sprayed evenly on the membrane, and then detection was performed. The results are shown in FIG. 3.

As shown in FIG. 3, the monoclonal antibodies 3D5 and 2H1 selected through the present invention were confirmed to show superior reactivity compared to the commercialized antibody (Size 35.3 kDa).

### Example 3. Manufacture of rapid diagnostic kit using the novel monoclonal antibodies

### 3-1. Antibody selection using rapid kit strip

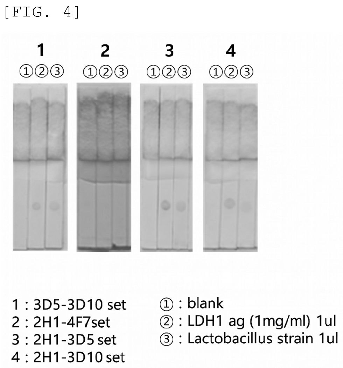

A rapid kit comprising the monoclonal antibodies selected in Example 2 was manufactured, and the detection ability for LDH1 was confirmed. More specifically, the monoclonal antibody candidates selected by the ELISA method were diluted to a concentration of 1 mg/ml each, and 1 µl was spotted on a nitrocellulose membrane. In addition, an antibody-gold nanoparticle conjugate was mixed with LDH1 (1 mg/ml) or a Lactobacillus strain, and the degree of color change at the spotting site was observed. The results are shown in FIG. 4.

As shown in FIG. 4, as a result of reacting the monoclonal antibodies according to the present invention in sets of two, it was confirmed that they did not bind to the Blank but reacted strongly with LDH1 and the Lactobacillus strain, and among them, set #3, the 2H1-3D5 set, was confirmed to show the best results.

### 3-2. Confirmation of detection ability of the finally selected antibodies on the rapid diagnostic kit

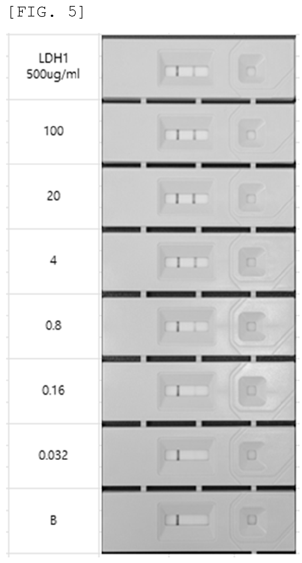

Using the rapid kit, the detection ability for the LDH1 antigen of the monoclonal antibodies 2H1 and 3D5 finally selected in 3-1 above was confirmed. Specifically, the 2H1 antibody was used as the capture antibody, and the 3D5 antibody was used as the detection antibody. Strips were prepared in 27x300 mm size by spotting the 3D5 antibody (test line) and anti-Nus A antibody (control line) on a nitrocellulose membrane. Then, the 2H1 antibody-gold nanoparticle conjugate and Nus A Protein-gold nanoparticles were dispensed onto a conjugate pad and dried to prepare it. The conjugate pad and sample pad were attached to the bottom of the nitrocellulose membrane, and the absorbent pad was attached to the top to manufacture the rapid kit. Then, samples prepared with LDH1 protein at different concentrations were placed in the sample injection part, and the degree of color change of the test line was observed. The results are shown in FIG. 5.

As shown in FIG. 5, it was confirmed that the two monoclonal antibodies, 2H1 and 3D5, can be applied to a rapid diagnostic kit for detecting *Lactobacillus plantarum* on the skin, and in particular, can be rapidly and accurately detected up to a level of about 0.032 µg/ml.

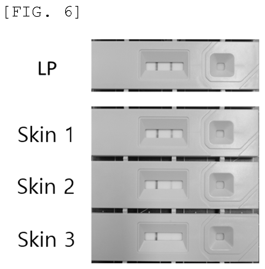

Next, an experiment was conducted to determine whether *Lactobacillus plantarum* could be detected using the rapid diagnostic kit developed above, using a sample prepared by scraping the actual skin epidermis where *Lactobacillus plantarum* is present. The results are shown in FIG. 6.

As shown in FIG. 6, it was confirmed that rapid diagnosis of *Lactobacillus plantarum* on actual skin is possible using the monoclonal antibodies 2H1 and 3D5 according to the present invention.

### Example 4. Sequence analysis of the novel monoclonal antibodies 2H1 and 3D5

Sequence analysis of the monoclonal antibodies 2H1 and 3D5, whose effects were confirmed in Example 3 above, was performed.

As a result, it was confirmed that the monoclonal antibody 2H1 consists of a heavy chain of SEQ ID NO: 7 comprising CDRs 1 to 3 of SEQ ID NOs: 1 to 3, and a light chain of SEQ ID NO: 8 comprising CDRs 1 to 3 of SEQ ID NOs: 4 to 6. In addition, the monoclonal antibody 3D5 was confirmed to consist of a heavy chain of SEQ ID NO: 15 comprising CDRs 1 to 3 of SEQ ID NOs: 9 to 11, and a light chain of SEQ ID NO: 16 comprising CDRs 1 to 3 of SEQ ID NOs: 12 to 14.

Through the above experimental results, it was confirmed that a rapid diagnostic kit capable of diagnosing skin conditions by directly determining the presence of *Lactobacillus plantarum* on the skin can be manufactured using the novel monoclonal antibodies 2H1 and 3D5 that specifically bind to the LDH1 protein of *Lactobacillus plantarum*.

**[Table 2]**

| **SEQ ID NO** | **Name** | **Sequence** |
|---|---|---|
| 1 | 2H1 Heavy Chain CDR1 | SFSGFSLSTSGMGVS |
| 2 | 2H1 Heavy Chain CDR2 | HIYWDDDKR |
| 3 | 2H1 Heavy Chain CDR3 | ARSGYYGSSSAWFAY |
| 4 | 2H1 Light Chain CDR1 | KSSQSLLYSSNQKNYLA |
| 5 | 2H1 Light Chain CDR2 | YWASTRES |
| 6 | 2H1 Light Chain CDR3 | QQYYSYPLT |
| 7 | 2H1 Heavy Chain | VQCQVKLEESGPGILQPSQTLSLTCSFSGFSLSTSGMGVS WIRQPSGKGLEWLAHIYWDDDKRYNPSLKSRLTISKDTS |
| | | RNQVFLKITSVDTADTATYYCARSGYYGSSSAWFAYWG QGTLVTVSAAKTTPPSVY |
| 8 | 2H1 Light Chain | GHHNVQTPSSLAVSVGEKVTMSCKSSQSLLYSSNQKNY LAWYQQKPGQSPKLLIYWASTRESGVPDRFTGSGSGTD FTLTISSVKAEDLAVYYCQQYYSYPLTFGAGTKLELKRAD AAPTVS |
| 9 | 3D5 Heavy Chain CDR1 | SFSGFSLSTSGMGVG |
| 10 | 3D5 Heavy Chain CDR2 | HIWWDDVKR |
| 11 | 3D5 Heavy Chain CDR3 | VRSLLAHYDGYYVIYAMDY |
| 12 | 3D5 Light Chain CDR1 | KSSQSLLYSSNQKNYLA |
| 13 | 3D5 Light Chain CDR2 | YWASTRES |
| 14 | 3D5 Light Chain CDR3 | QQYYSYPWT |
| 15 | 3D5 Heavy Chain | VQCEVQLQQSGPGILQSSQTLSLTCSFSGFSLSTSGMGV GWIRQPSGKGLEWLAHIWWDDVKRYNPALKSRLTISKD TSSSQVFLKIASVDTADTATYYCVRSLLAHYDGYYVIYAM DYWGQGTSVTVSSAKTTPPSV |
| 16 | 3D5 Light Chain | DIVLTQTPSSLAVSVGEKVTMSCKSSQSLLYSSNQKNYL AWCQQKPGQSPKLLIYWASTRESGVPDRFTGSGSGTDF TLTISSVKAEDLAVYYCQQYYSYPWTFGGGTKLEIKRADA APTVS |

While specific aspects of the present invention have been described in detail above, it will be apparent to those skilled in the art that these specific descriptions merely represent preferred embodiments and are not intended to limit the scope of the present invention. Therefore, the substantial scope of the present invention is defined by the appended claims and their equivalents.

## Claims

1. An antibody specific to the LDH1 protein of *Lactobacillus plantarum*, comprising a heavy chain variable region comprising heavy chain CDR1 represented by the amino acid sequence of SEQ ID NO: 1, heavy chain CDR2 represented by the amino acid sequence of SEQ ID NO: 2, and heavy chain CDR3 represented by the amino acid sequence of SEQ ID NO: 3; and a light chain variable region comprising light chain CDR1 represented by the amino acid sequence of SEQ ID NO: 4, light chain CDR2 represented by the amino acid sequence of SEQ ID NO: 5, and light chain CDR3 represented by the amino acid sequence of SEQ ID NO: 6.

2. The antibody of claim 1, wherein the antibody comprises a heavy chain variable region represented by the amino acid sequence of SEQ ID NO: 7.

3. The antibody of claim 1, wherein the antibody comprises a light chain variable region represented by the amino acid sequence of SEQ ID NO: 8.

4. An antibody specific to the LDH1 protein of *Lactobacillus plantarum*, comprising a heavy chain variable region comprising heavy chain CDR1 represented by the amino acid sequence of SEQ ID NO: 9, heavy chain CDR2 represented by the amino acid sequence of SEQ ID NO: 10, and heavy chain CDR3 represented by the amino acid sequence of SEQ ID NO: 11; and a light chain variable region comprising light chain CDR1 represented by the amino acid sequence of SEQ ID NO: 12, light chain CDR2 represented by the amino acid sequence of SEQ ID NO: 13, and light chain CDR3 represented by the amino acid sequence of SEQ ID NO: 14.

5. The antibody of claim 4, wherein the antibody comprises a heavy chain variable region represented by the amino acid sequence of SEQ ID NO: 15.

6. The antibody of claim 4, wherein the antibody comprises a light chain variable region represented by the amino acid sequence of SEQ ID NO: 16.

7. A polynucleotide encoding the antibody of any one of claims 1 to 6.

8. An expression vector comprising the polynucleotide of claim 7.

9. A host cell comprising the expression vector of claim 8.

10. A composition for detecting *Lactobacillus plantarum*, comprising the antibody of any one of claims 1 to 6.

11. A kit for detecting *Lactobacillus plantarum*, comprising the antibody of any one of claims 1 to 6.

12. The kit of claim 11, wherein the kit is an ELISA (Enzyme-linked immunosorbent assay) kit, a sandwich ELISA kit, a protein chip kit, or a rapid kit.

13. The kit of claim 11, wherein the kit comprises two types of antibodies, one type of antibody being a capture antibody and the other type of antibody being a detection antibody.

14. The kit of claim 11, wherein the kit is for diagnosing skin conditions.
